(19) Europäisches Patentamt
European Patent Office
Office européen des brevets

(11) Numéro de publication: 0 149 578 B1

(12) # FASCICULE DE BREVET EUROPÉEN

(45) Date de publication du fascicule du brevet: 25.01.89

(51) Int. Cl.⁴: **C 07 D 473/06**, C 07 D 473/04, C 07 D 473/08, C 07 D 473/12, C 07 D 473/10, A 61 K 31/52

(21) Numéro de dépôt: **85400072.6**

(22) Date de dépôt: **16.01.85**

(54) **Nouveaux dérivés de la xanthine, leur procédé de préparation et les compositions pharmaceutiques les renfermant.**

(30) Priorité: **17.01.84 FR 8400659**

(43) Date de publication de la demande: **24.07.85 Bulletin 85/30**

(45) Mention de la délivrance du brevet: **25.01.89 Bulletin 89/4**

(84) Etats contractants désignés: **AT BE CH DE FR GB IT LI LU NL SE**

(56) Documents cité:
**EP-A-0 021 103**
**EP-A-0 068 544**
**FR-M-5 086**

(73) Titulaire: **ADIR, 22, rue Garnier, F-92200 Neuilly- sur-Seine (FR)**

(72) Inventeur: **Regnier, Gilbert, avenue du Plessis, F-92290 Chatenay Malabry (FR)**
Inventeur: **Guillonneau, Claude, 21, Rue des Bergers, F-92140 Clamart (FR)**
Inventeur: **Duhault, Jacques, 14 bis rue Paul Demange, F-78290 Croissy S/Seine (FR)**
Inventeur: **Roman, François, 10 rue de Dieppe, F-92400 Courbevoie (FR)**

(74) Mandataire: **Reverbori, Marcelle, ADIR 22 Rue Garnier, F-92200 Neuilly- sur- Seine (FR)**

EP 0 149 578 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention a pour objet de nouveaux dérivés de la xanthine, leurs procédés de préparation et les compositions pharmaceutiques les renfermant.

Elle concerne particulièrement les dérivés de la xanthine de formule générale I:

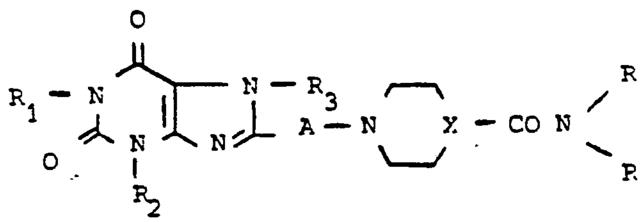

(I)

dans laquelle:

$R_1$ répresente un atome d'hydrogène ou un radical alcoyle renfermant de 1 à 5 atomes de carbone, en chaîne droite ou ramifiée,

$R_2$ représente un atome d'hydrogène, un radical alcoyle renfermant de 1 à 5 atomes de carbone, en chaîne droite ou ramfiée, et comportant éventuellement une double liaison, ou un radical benzyle;

$R_3$ représente un atome d'hydrogène ou un radical méthyle;

A représente une chaîne polyméthylénique $-(CH_2)_n-$ dans laquelle n représente un nombre entier variant de 1 à 4, éventuellement substituée, lorsque n est supérieur à 1, par un radical hydroxyle;

X représente un atome d'azote ou un radical 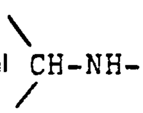 ;

et renferment de 1 à 5 atomes de carbone, ou forment ensemble une chaîne polyméthylénique ayant de 4 à 6 atomes de carbone, comportant éventuellement un atome d'oxygène ou de soufre, formant ainsi avec l'atome d'azote auquel ils sont liés un radical heterocylique renfermant un ou deux heteroatomes.

L'art antérieur est représenté par des xanthines substituées en position -1 ou -7 par une chaine amino-alkyle, et possédant des activités coronarodilatatrices (BSM 5086) antiischémiques myocardiques (EP 68544) ou antiallergiques (EP 21103). Les xanthines de la présente invention, substituées en position-8, possèdent d'une façon inattendue des propriétés antispasmodiques et psychostimulantes, et franchissant la barrière hemo-méningée, trouvent leur application thérapeutique comme anti-migraineux ou psychotropes.

La présente invention a pour objet le procédé de préparaton des dérivés de formule générale I caractérisé en ce que:

- l'on condense un dérivé halogéné de formule générale II

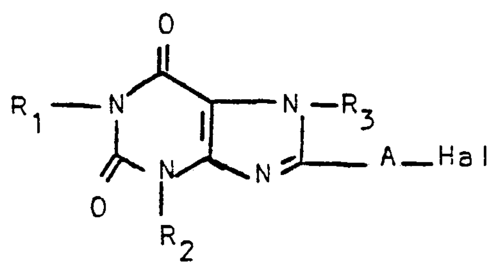

(II)

dans laquelle $R_1$, $R_2$, $R_3$ et A ont les significations précédemment définies, et Hal représente un atome de chlore ou de brome,

- avec un dérivé de formule générale III:

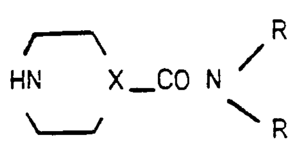

(III)

dans laquelle X et R ont les significations éconcées précédemment.

La condensation s'effectue de préférence dans un solvant choisi parmi les alcools renfermant jusqu'a 4 atomes de carbone, tel que par exemple le méthanol, l'éthanol, le propanol ou le butanol. Il est avantageux d'opérer à une température comprise entre 80 et 110°C, en présence d'un accepteur de l'hydracide formé au cours de la réaction. Cet accepteur peut être par exemple une amine tertiaire telle que la triéthylamine ou un excès du dérivé aminé de formule III utilisé pour la réaction.

La présente invention a aussi pour objet le procédé de préparation des dérivés de formule générale I,

caractérisé en ce que:
- l'on condense un derivé aminé de formule générale IV:

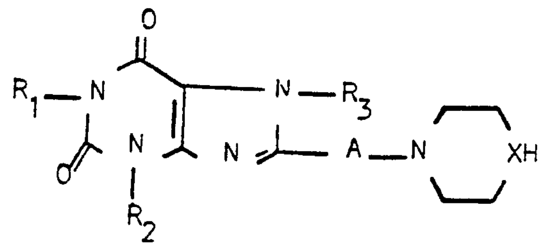

(IV)

dans laquelle $R_1$, $R_2$, $R_3$, A et X ont les significations précedemment définies,
- avec un dérivé halogéné de formule générale V:

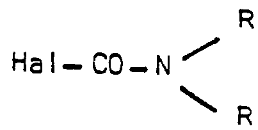

(V)

dans laquelle R a la signification enoncée précédemment et Hal représente un atome de chlorure ou de brome.

Il est particulièrement avantageux d'effectuer la condensation dans un solvant choisi parmi tetrahydrofuranne, le dioxane ou la pyridine, à une accepteur de l'hydracide formé au cours de la réaction. Comme accepteur, on peut utiliser par exemple la triéthylamine ou la pyridine.

Ces nouveaux dérivés (I) ainsi obtenus peuvent être transformés en sels d'addition avec les acides, sels qui font, à ce titre, partie de la présente invention. Comme acides utilisables pour la formation de ces sels, on peut citer, par exemple, dans la série minérale: les acides chlorhydrique, bromhydrique, sulfurique, phosphorique, et dans la série organique: les acides acétique, propionique, maléique, fumarique, tartrique, citrique, oxalique, benzoïque, méthane sulfonique et iséthionique.

Ces nouveaux dérivés (I) peuvent être purifiés par des méthodes physiques telles que cristallisation, chromatographie ou chimiques telles que formation de sels d'addition avec des acides et décomposition de ces sels par des agents alcalins.

Les matières premières de formule générale II ont été préparées selon les techniques décrites dans la demande de brevet français publiée sous le n° 2 531 085 le 3 février 1984.

Les matières premières de formule générale IV ont été préparées par débenzylation au moyen d'hydrogène, en présence de charbon palladié sous pression des dérivés benzyles correspondants, eux-mêmes préparés selon la demande de brevet français publiée sous le n° 2 531 085 le 3 février 1984.

Les dérivés de formule générale I et leurs sels physiologiquement tolérables possèdent des propriétés pharmacologiques et thérapeutiques intéressantes, notamment des propriétés anti-migraineuse et psychotonique centrale.

Les dérivés étudies présentent une activité spasmolytique mise en évidence in vitro sur préparation isolée de taenia coli de cobaye (FERRARI M. et CARPENEDOF; Arch. Int. Pharmacol (1968) 174, 223). Ces substances inhibent la contraction qui accompagne la dépolarisation de membrane provoquée par les ions $K^+$ et la contraction provoquée par le $Ca^{++}$ extracellulaire. Les doses efficaces moyennes ($DE_{50}$) varient selon les produits de $2.10^{-5}$M à $8.10^{-4}$M.

D'autre part, ces composés inhibent l'activité enzymatique de la phospho-diestérase responsable de la dégradation du 3',5'-adénosine monophosphate cyclique. L'accumulation intracellulaire de cette substance peut également expliquer la relaxation des fibres musculaires lisses. Lorsque la source enzymatique est un broyat de cerveau de rat, la concentration inhibitrice moyenne ($CI_{50}$) est située entre 1,5 - $10^{-5}$M et $1.10^{-4}$M (NAIR K.G., Biochem (1966), 5, 150). Dans les mêmes conditions expérimentales, la théophylline à une $CI_{50}$ de $1,2.10^{-4}$M.

Ces dérivés (I) sont peu toxiques; leur dose léthale moyenne ($DL_{50}$) est supérieure à 800 mg/kg per os.

Il ont, de plus, un effet stimulant du système nerveux central dès la dose de 50 mg/kg per os chez le cobaye.

Un effet analgésique (selon la méthode de la plaque chauffante, WOOLFE G. et Mc DONALD A.D., J.P.E.T. (1964) 80, 300) a pu être observé chez la souris NMRI pesant 26 g, après administration orale de certains dérivés (I) à la dose de 50 mg/kg. Cette activité à egalement été mise en évidence par le test à la phénylbenzoquinone, chez la souris CD. A la dose de 50 mg/kg per os, les composés de formule générale I ont une activité antalgique supérieure ou égale à celle de l'aspirine.

Ces propriétés analgésiques, jointes aux effets sur la contraction des fibres lisses permettent l'utilisation des dérivés de formule générale I dans le traitement de la migraine, en particulier pendant la période initiale qui comporte une phase de vaso-constriction.

Ces dits composés peuvent également être utilisés dans le traitement de l'asthénie, du fait de leur activité psychotonique centrale.

La présente invention à également pour objet les compositions pharmaceutiques contenant comme principe actif un dérivé de formule générale I, ou un de ses sels physiologiquement tolérables, mélangé ou associé à un

excipient pharmaceutique approprié.

Les compositions pharmaceutiques ainsi obtenues sont présenteées avantageusement sous des formes diverses telles que, par exemple, comprimés, dragées, gélules, glossettes ou préparations galéniques appropriées pour une administration sublinguale, suppositoires, solutions injectables ou buvables ainsi que sous des formes adaptées à l'administration par aérosol.

Les exemples suivants donnés à titre non limitatif, illustrent l'invention. Les points de fusion, sauf mention contraire, ont été déterminés à la platine chauffante de Kofler.

**Exemple 1**

Triméthyl-1,3,7 [(diéthylaminocarbonyl-4-pipérazino)-3 propyl]-8 xanthine.

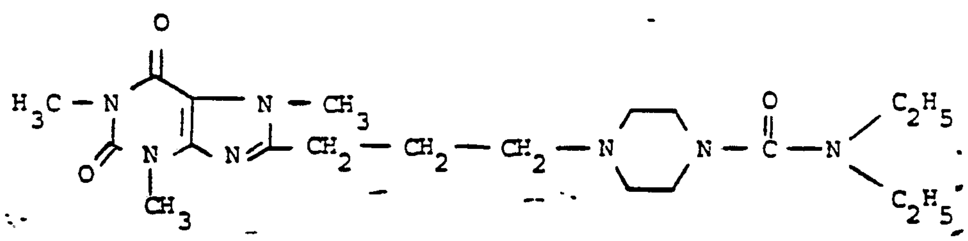

**Première méthode**

On chauffe pendant 20 heures à reflux une solution de 21,1 g de triméthyl-1,3,7 (bromo-3 propyl)-8 xanthine fondant à 130°C et de 24,7 g de diéthylcarbamido-1 pipérazine (Eb/0,15 mm Hg = 116-122°C)dans 530 ml d'éthanol. Ensuite, on evapore le solvant sous pression réduite et reprend le résidu par 200 ml de $NaHCO_3$ 10 % et 200 ml de $CH_2Cl_2$. On décante et sèche la solution organique sur sulfate de magnésium. On évapore le solvant et purifie le mélange par chromatographie flash sur 1200 g de silice (0,063-0,2) en éluant avec un mélange $CH_2Cl_2$ -$CH_3OH$ (92,5/7,5). Après évaporation des éluats, on obtient 17,3 g de triméthyl-1,3,7 [(diéthylaminocarbonyl-4 pipérazino)-3 propyl] -8 xanthine, sous forme de cristaux blanc crème fondant à 112°C.

Les matières premières utilisées dans ce procédé ont été préparées essentiellement selon les techniques décrites dans la demande de brevet français publiée sous le n° 2 531 085 le 3 février 1984.

**Deuxième méthode**

A une solution de 8 g de triméthyl-1,3,7 (pipérazino-3 propyl)-8 xanthine (huile) dans 100 ml de tétrahydrofuranne anhydre contenant 2,5 g de triéthylamine; on ajoute en 15 minutes à la température de 10°C, 3,5 g de chlorure de diéthyl carbamyle. Après une heure à température ambiante, on chauffe le mélange pendant 1 heure à 50°C, puis refroidit et évapore le solvant sous pression réduite. Le résidu est traité comme dans la première méthode. On obtient 7 g de cristaux de triméthyl-1,3,7 [(diéthylaminocarbonyl-4 pipérazino)-3 propyl] -8 xanthine fondant à 112°C.

La trimethyl-1,3,7 (pipérazino-3 propyl)-8 xanthine de départ a été préparée par débenzylation par l'hydrogène en présence de charbon palladié, sous une pression d'environ $3.10^6$Pa, de la triméthyl-1,3,7 [(benzyl-4 piperazino)-3 propyl] -8 xanthine, elle-même préparée comme mentionné dans la demande de brevet français publiée sous le n° 2 531 085 3 février 1984.

**Exemples 2 à 15**

Les dérivés suivants ont été préparés selon les méthodes décrites dans l'exemple 1.

2) Diméthyl-1,7 isobutyl-3 [(diéthylaminocarbonyl-4 pipérazino)-3 propyl]-8 xanthine, P.F.: 118°C (chlorure de méthylène).

3) Diméthyl-1,7 n.propyl-3 [(diéthylaminocarbonyl-4 pipérazino)-3 propyl]-8 xanthine, P.F. du fumarate correspondant: 161°C (n.propanol-éther)

4) Ethyl-1 isobutyl-3 méthyl-7[(diéthylaminocarbonyl-4 pipérazino)-3 propyl)]-8 xanthine,P.F.: 101°C (chlorure

de méthylène)

5) Méthyl-1 isobutyl-3 (diéthylamino carbonyl-4 pipérazinométhyl)-8 xanthine, dont le chlorhydrate est un produit amorphe.

6) Diméthyl-1,3 [(diéthylaminocarbonyl-4 pipérazino)-2 éthyl]-8 xanthine, P.F. (capillaire): 152-153°C chlorure de méthylène).

7) Triméthyl-1,3,7 [(diéthylamino carbonyl-4 pipérazino)-2 éthyl)]-8 xanthine, P.F. (capillaire) du difumarate correspondant: 185 - 186°C (n. propanol-éther)

8) diméthyl-1,3 [(diéthylamino carbonyl-4 pipérazino)-3 propyl)]-8 xanthine, P.F.: 177°C (chlorure de méthylène).

9) Benzyl-3 [(diéthylamino carbonyl-4 pipérazino)-2 éthyl)] -8 xanthine, P.F.: 170°C (acétate d'éthyle).

10) Triméthyl-1,3,7 [(pipéridino carbonyl-4 pipérazino)-3 propyl]-8 xanthine, P.F.: 152°C (éthanol).

11) Triméthyl-1,3,7 [(morpholino carbonyl-4 pipérazino)-3 propyl]-8 xanthine, P.F.: 162°C (chlorure de méthylène).

12) Triméthyl-1,3,7 [(diéthylamino carbonylamino-4 pipéridino)-3 propyl]-8 xanthine, P.F.: 156°C (chlorure de méthylène).

13) Diméthyl-3,7 [(diéthylamino carbonyl-4 pipérazino)-2 hydroxy-1 éthyl]-8 xanthine.

14) Triméthyl-1,3,7 [(diéthylamino carbonyl-4 piperazino)-2 hydroxy-1 éthyl]-8 xanthine.

15) Diméthyl-3,7 [(diéthylamino carbonyl-4 pipérazino)-2 éthyl]-8 xanthine.

Les matières premières, utilisées pour la synthèse des composés ci-dessus, autres que celles décrites dans la demande de brevet français n° 8 213 155 du 28 juillet 1982 sont les suivantes:

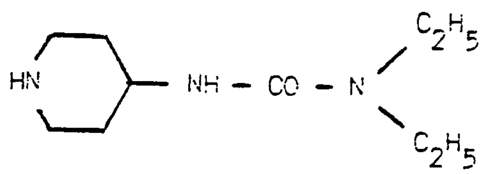

; P.F. : 120-130°C

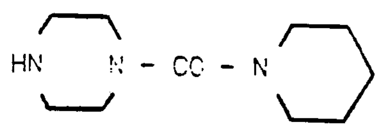

; P.F. : 72°C

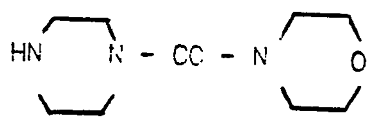

; P.F. : 82°C

et les dérivés de formule:

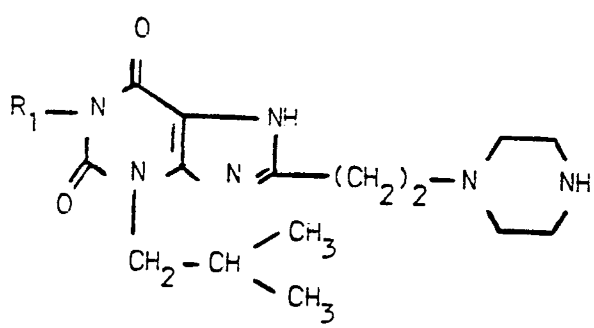

$R_1$ = H, P.F. (capillaire) du dichlorhydrate: 194-198°C

$R_1$ = $CH_3$, P.F. (capillaire) du dichlorhydrate: 165-175°C.

préparés par débenzylation, sous hydrogène, en présence de charbon palladié sous une pression de $10^7$ Pa, des dérivés benzylés correspondants.

**Revendications** pour les etats contractants: BE, CH, DE, FR, GB, IT, LI, LU, NL, SE

1. Les dérivés de la xanthine de formule générale I:

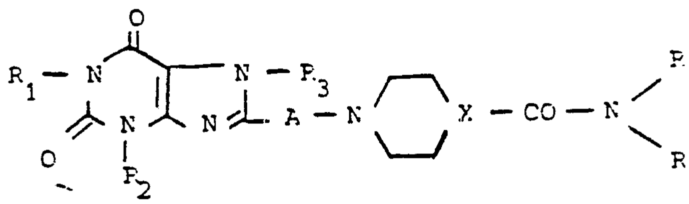

(I)

dans laquelle:

$R_1$ représente un atome d'hydrogène ou un radical alcoyle renfermant de 1 à 5 atomes de carbone, en chaine droite ou ramifiée;

$R_2$ représente un atome d'hydrogène, un radical alcoyle renfermant de 1 à 5 atomes de carbone en chaine droite ou ramifiée, et comportant éventuellement une double liaison, ou un radical benzyle;

$R_3$ représente un atome d'hydrogène ou un radical méthyle;

A représente une chaine polymétylénique $-(CH_2)_n-$ dans laquelle n représente un nombre entier variant de 1 à 4, éventuellement substitué lorsque n est supérieur à 1 par un radical hydroxyle;

X représente un atome d'azote ou un radical 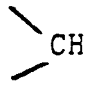 CH - NH - ;

et les substituants R représentent chacun un radical alcoyle renfermant de 1 à 5 atomes de carbone, ou forment ensemble une chaine polyméthylénique ayant de 4 à 6 atomes de carbone comportant éventuellement un atome d'oxygène ou de soufre, formant ainsi avec l'atome d'azote auquel ils sont liés un radical hétérocyclique renfermant un ou deux hétéro-atomes.

2. Les sels des composés de la revendication 1 avec des acides appropriés.

3. Les sels selon la revendication 2 qui sont physiologiquement tolérables.

4. La triméthyl-1,3,7,[(diéthylaminocarbonyl-4 pipérazino)-3 propyl)]-8 xanthine.

5. La diméthyl-1,7 isobutyl-3 [(diéthylaminocarbonyl-4 pipérazino)-3 propyl)]-8 xanthine.

6. La triméthyl-1,3,7,[(diéthylaminocarbonylamino-4 pipéridino)-3 propyl)]-8 xanthine.

7. Le procédé de préparation des composés de la revendication 1, caractérisé en ce que l'on condense:

- un dérivé halogéné de formule générale II:

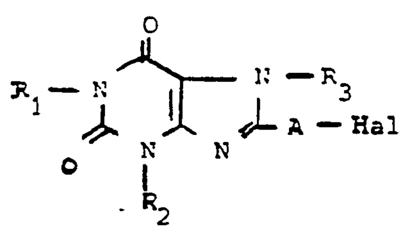

(II)

dans laquelle $R_1$, $R_2$, $R_3$ et A ont les significations définies dans la revendication 1 et Hal représente un atome de chlore ou de brome,

- avec un dérivé de formule générale III:

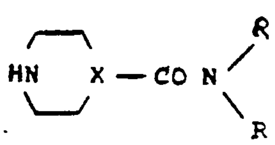

(III)

dans laquelle X et R ont les significations éconcées dans la revendication 1.

8. Le procédé selon la revendication 7, caractérisé en ce que l'on effectue la condensation dans un solvant choisi parmi les alcools renfermant jusqu'à 4 atomes de carbone, à une température comprise entre 80 et 110°C, en présence d'un accepteur de l'hydracide formé au cours de la réaction.

9. Le procédé de préparation des composés de la revendication 1 caractérisé en ce que l'on condense:

- un dérivé aminé de formule générale IV:

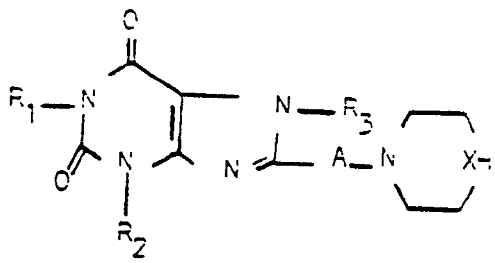

(IV)

dans laquelle $R_1$, $R_2$, $R_3$, A et X ont les significations enoncées dans la revendication 1,

- avec un dérivé halogéné de formule générale V:

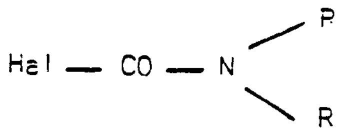

(V)

dans laquelle R est tel que défini dans la revendication 1 et Hal représente un atome de chlore ou de brome.

10. Le procédé selon la revendication 9, caractérisé en ce que l'on effectue la condensation dans un solvant choisi parmi le tétrahydrofuranne, le dioxane et la pyridine, à une température comprise entre 20 et 60°C, en présence d'un accepteur de l'hydracide formé au cours de la réaction.

11. Les compositions pharmaceutiques contenant comme principe actif un composé selon les revendications 1 ou 3 à 6, avec les excipients pharmaceutiques appropriés.

12. Les compositions pharmaceutiques selon la revendication 11 utilisées pour le traitement de la migraine et de l'asthénie.

**Revendication** pour l'etat contractant AT

Procédé de préparation des dérivés de la xanthine de formule générale I:

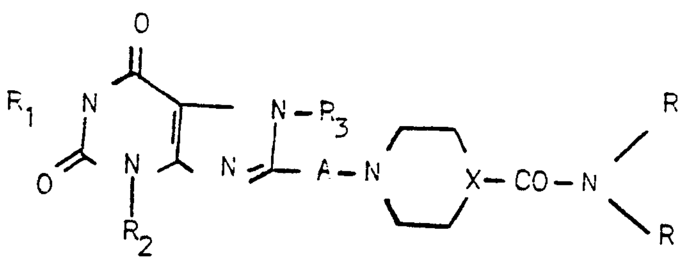

(I)

dans laquelle:

$R_1$ représente un atome d'hydrogène ou un radical alcoyle renfermant de 1 à 5 atomes de carbone, en chaine droite ou ramifiée;

$R_2$ représente un atome d'hydrogène, un radical alcoyle renfermant de 1 à 5 atomes de carbone en chaine droite ou ramifiée, et comportant éventuellement une double liaison ou un radical benzyle;

$R_3$ représente un atome d'hydrogène ou un radical méthyle;

A représente une chaine polyméthylénique $-(CH_2)_n-$ dans laquelle n représente un nombre entier variant de 1 à 4, éventuellement substitué lorsque n est superieur à 1 par un radical hydroxyle;

X représente un atome d'azote ou un radical 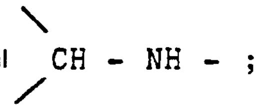

et les substituants R représentent chacun un radical alcoyle renfermant de 1 à 5 atomes de carbone, ou forment ensemble une chaine polyméthylénique ayant de 4 à 6 atomes de carbone comportant éventuellement un atome d'oxygène ou de soufre, formant ainsi avec l'atome d'azote auquel ils sont liés un radical hétérocyclique renfermant un ou deux hétéro-atomes;

et de leurs sels d'addition avec des acides appropriés,

caractérisé en ce que l'on condense soit:

a) un dérivé halogéné de formule générale II:

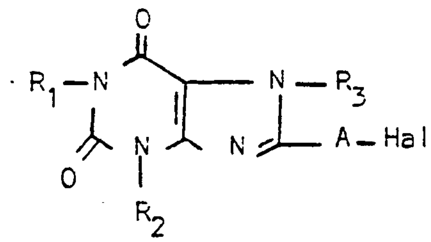

(II)

dans laquelle $R_1$, $R_2$, $R_3$ et A ont les significations définies précédemment et Hal représente un atome de chlore ou de brome,

- avec un dérivé de formule generale III:

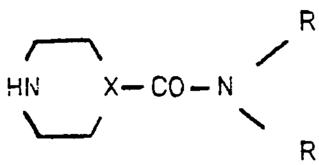

(III)

dans laquelle X et R ont les significations énoncées précédemment;

soit:

- un dérivé aminé de formule générale IV:

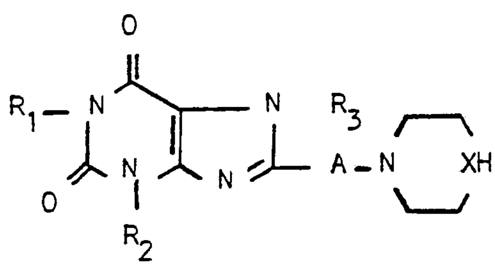

(IV)

dans laquelle $R_1$, $R_2$, $R_3$, A et X ont les significations énoncées précédemment,

- avec un dérivé halogéné de formule générale V:

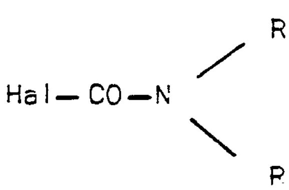

(V)

dans laquelle R et Hal sont tels que définis précédemment,

et si on le désire, on traite les dérivés ainsi obtenus, avec des acides appropriés, pour donner les sels d'addition correspondants.

**Patentansprüche** für die Vertragsstaaten: BE, CH, DE, FR, GB, IT, LI, LU, NL, SE

1. Xanthinderivate der allgemeinen Formel I:

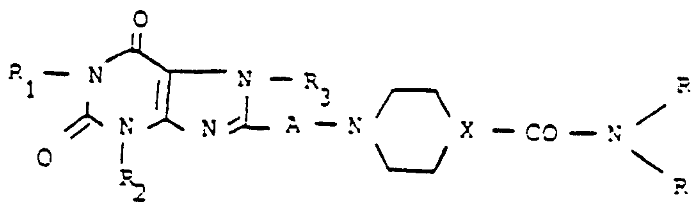

(I)

in der

$R_1$ ein Wasserstoffatom oder eine geradkettige oder verzweigte Alkylgruppe mit 1 bis 5 Kohlenstoffatomen;

$R_2$ ein Wasserstoffatom, eine geradkettige oder verzweigte Alkylgruppe mit 1 bis 5 Kohlenstoff-atomen, die gegebenenfalls eine Doppelbindung aufweisen kann, oder eine Benzylgruppe;

$R_3$ ein Wasserstoffatom oder eine Methylgruppe;

A eine Polymethylenkette der Formel $-(CH_2)_n-$ worin n für eine ganze Zahl mit einem Wert von 1 bis 4 steht, die dann, wenn n größer als 1 ist, gegebenenfalls durch eine Hydroxylgruppe substituiert ist;

X ein Stickstoffatom oder eine Gruppe der Formel $>CH-NH-$;

und die Substituenten R jeweils Alkylgruppen mit 1 bis 5 Kohlenstoffatomen oder gemeinsam eine Polymethylenkette mit 4 bis 6 Kohlenstoffatomen, die gegebenenfalls ein Sauerstoffatom oder ein Schwefelatom enthalten kann, und in dieser Weise gemeinsam mit dem Stickstoffatom, an das sie gebunden sind, einen ein oder zwei Heteroatome enthaltenden heterocyclischen Rest bilden;

bedeuten.

2. Die Salze der Verbindungen von Anspruch 1 mit geeigneten Säuren.
3. Die Salze nach Anspruch 2, die physiologisch verträglich sind.
4. 1,3,7-Trimethyl-8-(3-(4-diethylaminocarbonyl-piperazino)-propyl]-xanthin.
5. 1,7-Dimethyl-3-isobutyl-8-(3-(4-diethylaminocarbonyl-piperazino)-propyl]-xanthin.
6. 1,3,7-Trimethyl-8-(3-(4-diethylaminocarbonyl-piperadino-)-propyl]-xanthin.
7. 1,3,7-Trimethyl-8-(3-(4-diethylaminocarbonylamino-piperidino)-propyl]-xanthin.
7. Verfahren zur Herstellung der Verbindungen nach Anspruch 1, dadurch gekennzeichnet, daß man ein Halogenderivat der allgemeinen Formel II

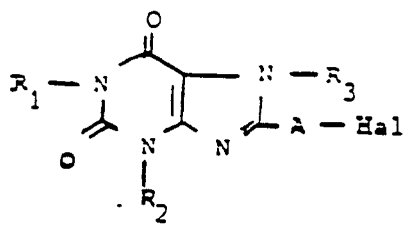

(II)

in der $R_1$, $R_2$, $R_3$ und A die in Anspruch 1 angegebenen Bedeutungen besitzen und Hal ein Chloratom oder ein Bromatom bedeutet,

mit einem Derivat der allgemeinen Formel III

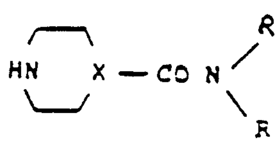

(III)

in der X und R die in Anspruch 1 angegebenen Bedeutungen besitzen, kondensiert.

8. Verfahren nach Anspruch 7, dadurch gekennzeichnet, daß man die Kondensation in einem Lösungsmittel ausgewählt aus Alkoholen mit bis zu 4 Kohlenstoffatomen, bei einer Temperatur zwischen 80 und 110°C in Gegenwart eines Akzeptors für die im Verlaufe der Reaktion gebildete Wasserstoffsäure bewirkt.

9. Verfahren zur Herstellung der Verbindungen nach Anspruch 1, dadurch gekennzeichnet, daß man ein Aminderivat der allgemeinen Formel IV

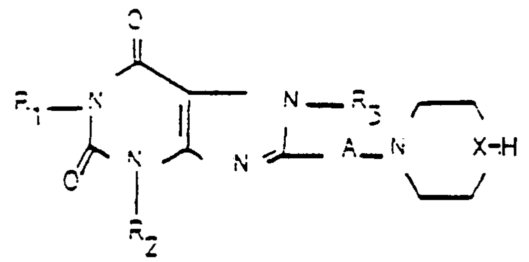

(IV)

in der $R_1$, $R_2$, $R_3$, A und X die in Anspruch 1 angegebenen Bedeutungen besitzen, mit einem Halogenderivat der allgemeinen Formel V

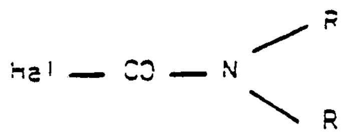

(V)

worin R die in Anspruch 1 angegebenen Bedeutungen besitzt und Hal ein Chlor- oder Bromatom bedeutet, kondensiert.

10. Verfahren nach Anspruch 9, dadurch gekennzeichnet, daß man die Kondensation in einem aus Tetrahydrofuran, Dioxan und Pyridin ausgewählten Lösungsmittel bei einer Temperatur zwischen 20 und 60°C in Gegenwart eines Akzeptors für die im Verlaufe der Reaktion gebildete Wasserstoffsäure bewirkt.

11. Pharmazeutische Zubereitungen enthaltend als Wirkstoff eine Verbindung nach einem der Ansprüche 1 oder 3 bis 6 mit geeigneten pharmazeutischen Trägern.

12. Phannazeutische Zubereitungen nach Anspruch 11 in einer zur Behandlung der Migräne und der Asthenie geeigneten Form.

**Patentanspruch** für den Vertagsstaaten AT

Verfahren zur Herstellung von Xanthinderivaten der allgemeinen Formel I

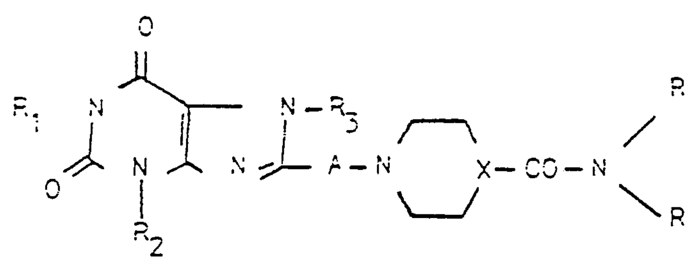

(I)

in der

$R_1$ ein Wasserstoffatom oder eine geradkettige oder verzweigte Alkylgruppe mit 1 bis 5 Kohlenstoffatomen;

$R^2$ ein Wasserstoffatom, eine geradkettige oder verzweigte Alkylgruppe mit 1 bis 5 Kohlenstoffatomen, die gegebenenfalls eine Doppelbindung aufweisen kann, oder eine Benzylgruppe;

$R^3$ ein Wasserstoffatom oder eine Methylgruppe;

A eine Polymethylenkette der Formel $-(CH_2)_n-$ worin n für eine ganze Zahl mit einem Wert von 1 bis 4 steht, die dann, wenn n größer als 1 ist, gegebenenfalls durch eine Hydroxylgruppe substituiert ist;

X ein Stickstoffatom oder eine Gruppe der Formel $>CH-NH-$;

und die Substituenten R jeweils Alkylgruppen mit 1 bis 5 Kohlenstoffatomen oder gemeinsam eine Polymethylenkette mit 4 bis 6 Kohlenstoffatomen, die gegebenenfalls ein Sauerstoff-atom oder ein Schwefelatom enthalten kann, und in dieser Weise gemeinsam mit dem Stickstoffatom, an das sie gebunden sind, einen ein oder zwei Heteroatome enthaltenden heterocyclischen Rest bilden,

bedeuten;

und deren Säureadditionssalzen mit geeigneten Säuren,

dadurch gekennzeichnet, daß man entweder

a) ein Halogenderivat der allgemeinen Formel II

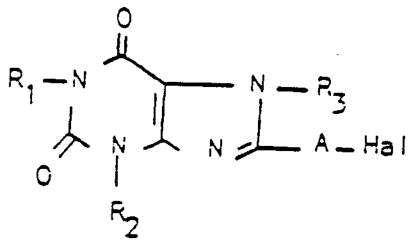

(II)

in der $R_1$, $R_2$, $R_3$ und A die oben angegebenen Bedeutungen besitzen und Hal ein Chloratom oder ein Bromatom darstellt,

mit einem Derivat der allgemeinen Formel III

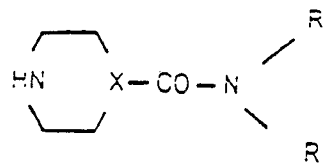

(III)

in der X und R die oben angegebenen Bedeutungen besitzen, kondensiert; oder

b) ein Aminderivat der allgemeinen Formel IV

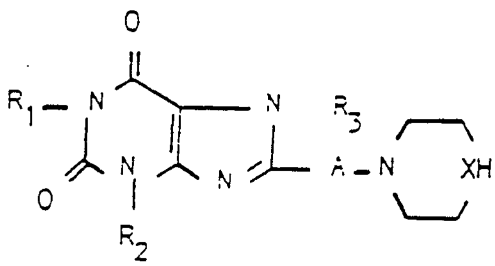

(IV)

in der $R_1$, $R_2$, $R_3$, A und X die oben angegebenen Bedeutungen besitzen, mit einem Halogenderivat der allgemeinen Formel V

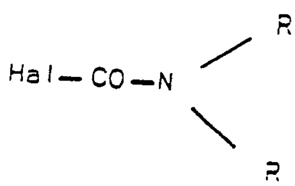

(V)

in der R und Hal die oben angegebenen Bedeutungen besitzen, kondensiert und gewünschtenfalls die in dieser Weise erhaltenen Derivate mit geeigneten Säuren zur Bildung der entsprechenden Additionssalze behandelt.

**Claims** for the Contracting States: BE, CH, DE, FR, GB, IT, LI, LU, NL, SE

1. Xanthine derivatives of the general formula I:

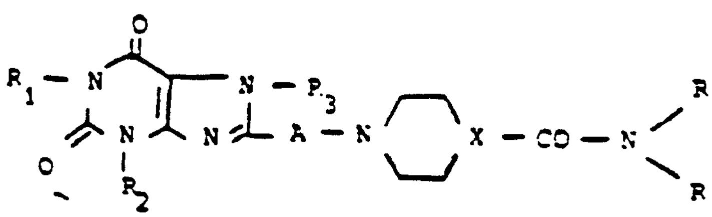

(I)

in which

$R_1$ represents a hydrogen atom or a straight-chain or branched alkyl radical containing from 1 to 5 carbon

atoms,

$R_2$ represents a hydrogen atom, a straight-chain or branched alkyl radical having from 1 to 5 carbon atoms which may contain a double bond, or a benzyl radical,

$R_3$ represents a hydrogen atom or a methyl radical,

A represents a polymethylene chain $-(CH_2)_n-$ in which n is an integer of from 1 to 4, which may be substituted, when n is greater than 1, by a hydroxy group,

X represents a nitrogen atom or a 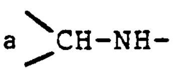 group, and each of the substituents R represents an alkyl radical containing from 1 to 5 carbon atoms, or the substituents R together form a polymethylene chain having from 4 to 6 carbon atoms which may contain an oxygen atom or sulphur atom, thus forming, with the nitrogen atom to which the substituents R are bonded, a heterocyclic radical containing one or two hetero atoms.

2. The salts according to claim 1 with appropriate acids.

3. The salts according to claim 2 that are physiologically tolerable.

4. 1,3,7-Trimethyl-8-[3-(4-diethylaminocarbonylpiperazino)-propyl]-xanthine.

5. 1,7-Dimethyl-3-isobutyl-8-[3-(4-diethylaminocarbonylpiperazino)-propyl]-xanthine.

6. 1,3,7-Trimethyl-8-[3-(4-diethylaminocarbonylaminopiperidino)-propyl]-xanthine.

7. Process for the preparation of compounds according to claim 1, characterised in that a halogen derivative of the general formula II:

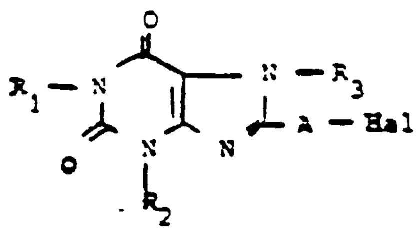

(II)

in which $R_1$, $R_2$, $R_3$ and A have the meanings given in claim 1 and Hal represents a chlorine or bromine atom is condensed with a derivative of the general formula III:

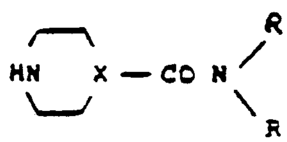

(III)

in which X and R have the meanings given in claim 1.

8. A process according to claim 7, characterised in that the condensation is carried out in a solvent selected from alcohols containing up to 4 carbon atoms at a temperature between 80 and 110°C in the presence of a hydracid acceptor formed during the reaction.

9. A process for the preparation of the compounds of claim 1, characterised in that an amine derivative of the general formula IV:

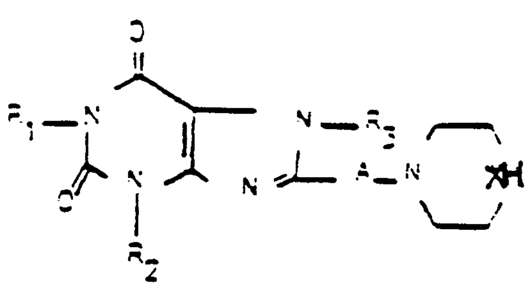

(IV)

in which $R_1$, $R_2$, $R_3$, A and X have the meanings given in claim 1, is condensed with a halogen derivative of the general formula V:

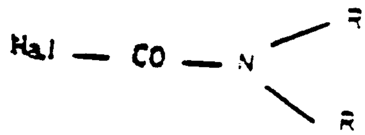

(V)

in which R is as defined in claim 1 and Hal represents a chlorine or bromine atom.

10. A process according to claim 9, characterised in that the condensation is carried out in a solvent selected from tetrahydrofuran, dioxan and pyridine at a temperature between 20 and 60°C in the presence of an acceptor of the hydracid formed during the reaction.

11. Pharmaceutical compositions containing as active ingredient a compound according to claims 1 or 3 to 6, with appropriate pharmaceutical excipients.

12. Pharmaceutical compositions according to claim 11 used for the treatment of migraine and asthenia.

**Claim** for the Contracting State: AT

1. Process for the preparation of xanthine derivatives of the general formula I:

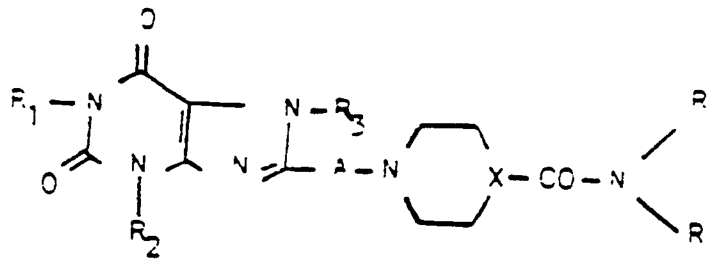

(I)

in which

$R_1$ represents a hydrogen atom or a straight-chain or branched alkyl radical containing from 1 to 5 carbon atoms,

$R_2$ represents a hydrogen atom, a straight-chain or branched alkyl radical having from 1 to 5 carbon atoms which may contain a double bond, or a benzyl radical,

$R_3$ represent a hydrogen atom or a methyl radical,

A represents a polymethylene chain $-(CH_2)_n-$ in which $\underline{n}$ is an integer of from 1 to 4, which may be substituted, when $\underline{n}$ is greater than 1, by a hydroxy group,

X represents a nitrogen atom or a $>CH-NH-$ group, and

each of the substituents R represents an alkyl radical containing from 1 to 5 carbon atoms, or the substituents R together form a polymethylene chain having from 4 to 6 carbon atoms which may contain an oxygen atom or sulphur atom, thus forming, with the nitrogen atom to which the substituents R are bonded, a heterocyclic radical containing one or two hetero atoms, and the addition salts thereof with appropriate acids, characterised in that either:

a) a halogen derivative of the general formula II:

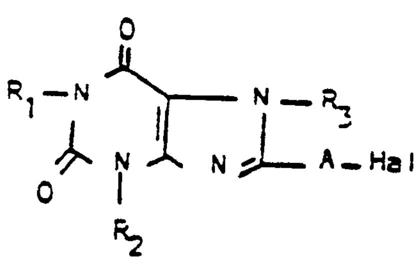

(II)

in which $R_1$, $R_2$, $R_3$ and A have the meanings given above and Hal represents a chlorine or bromine atom is condensed with a derivative of the general formula III:

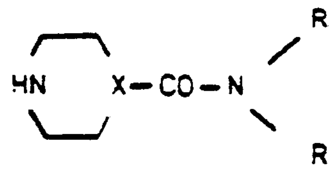

(III)

in which X and R have the meanings given in claim 1,
or
an amine derivative of the general formula IV:

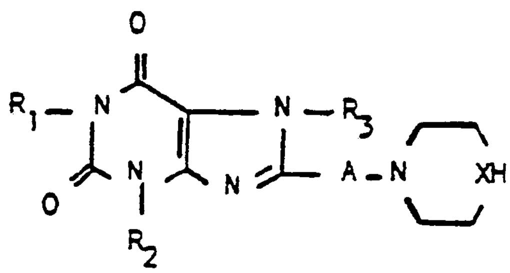

(IV)

in which $R_1$, $R_2$, $R_3$, A and X have the meanings given above, is condensed with a halogen derivative of the general formula V:

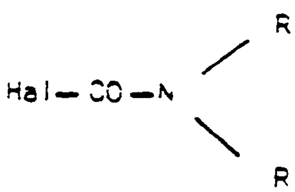

(V)

in which R and Hal are as defined above and, if desired, the so-obtained derivatives are treated with appropriate acids to yield corresponding addition salts.